(19) Europäisches Patentamt
European Patent Office
Office européen des brevets

(11) EP 2 770 058 A1

(12) EUROPEAN PATENT APPLICATION

(43) Date of publication:
**27.08.2014 Bulletin 2014/35**

(21) Application number: **13305214.2**

(22) Date of filing: **26.02.2013**

(51) Int Cl.:
***C12N 15/115*** *(2010.01)* ***C12Q 1/68*** *(2006.01)*
***G01N 33/53*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Université de Perpignan**
**66860 Perpignan (FR)**

(72) Inventors:
- **Marty, Jean-Louis**
**66400 Ceret (FR)**
- **Prieto-Simon, Beatriz**
**08240 Manresa (ES)**

(74) Representative: **Regimbeau**
**20, rue de Chazelles**
**75847 Paris Cedex 17 (FR)**

# (54) Ligand and method for detection of okadaic acid

(57) The present invention relates to isolated nucleic acid compounds that bind with high affinity and specificity to okadaic acid. Okadaic acid (OA) is a lipophilic phycotoxin responsible for human intoxication after seafood ingestion. The present invention also relates to the use of said nucleic acid compounds for detecting the presence and for quantitatively assaying okadaic acid in a sample. The present invention also relates to devices and kits comprising said nucleic acid compounds usable for the detection of okadaic acid, and to methods allowing okadaic acid detection in a sample.

EP 2 770 058 A1

SEQUENCE LISTING

<110> Universite de Perpignan

<120> Detection of Okadaic Acid

<130> 70348D31742

<160> 8

<170> PatentIn version 3.5

<210> 1
<211> 63
<212> DNA
<213> artificial

<220>
<223> Sequence Aptamere N°1

<400> 1
cgggtgtggc gggtgggtgg attttgaggt ctatggtggt gttttgagag gggtttctgg 60

gtc 63

<210> 2
<211> 63
<212> DNA
<213> artificial

<220>
<223> Sequence Aptamere N°2

<400> 2
gggagtggtg ggtgggtgtt taagatgggg tttatgaatg ttgcgtcagc cctctctcga 60

gtc 63

<210> 3
<211> 63
<212> DNA
<213> artificial

<220>
<223> Sequence Aptamere N°3

<400> 3
ccgggtgggt gggtgtggtc ttgtatttga ttatgtctgt cggcgctttt tggccccttc 60

gtt 63

<210> 4
<211> 63
<212> DNA
<213> artificial

<220>
<223> Sequence Aptamere N°4

<400> 4

aaggcaatgg gactatcggg acggaaagtt gttttaggca gagctggtcc taatctaccc 60

atg 63

<210> 5
<211> 63
<212> DNA
<213> artificial

<220>
<223> Sequence Aptamere N°5

<400> 5
cgtgctgtcg tgcgagggcc aggggatacc aggtagatta tagtttttgg gggcttttgg 60

tgg 63

<210> 6
<211> 62
<212> DNA
<213> artificial

<220>
<223> Sequence Aptamere N°6

<400> 6
ggctctgggc gggggttgg gcagagagcc tatgtcacct gtcgggttcc tctattcttc 60

gg 62

<210> 7
<211> 63
<212> DNA
<213> artificial

<220>
<223> Sequence Aptamere N°7

<400> 7
tcacaaccgg tgtggtagtt atcgttgggg gctatgtcat gtttattggt attgtgattg 60

gcg 63

<210> 8
<211> 61
<212> DNA
<213> artificial

<220>
<223> Sequence Aptamere N°8

<400> 8
agcgagggtt tacaatggtg ggcttgatgg tcttgggtcg tcaggcttgt tcggtttggg 60

t 61

## Description

The present invention relates to isolated nucleic acid compounds that bind with high affinity and specificity to okadaic acid. The present invention also relates to the use of said nucleic acid compounds for detecting the presence and for quantitatively assaying okadaic acid in a sample. The invention also relates to devices and kits comprising said nucleic acid compounds usable for the detection of okadaic acid, and to methods for okadaic acid detection in a sample.

Okadaic acid (OA) is a lipophilic phycotoxin responsible for human intoxication after seafood ingestion. Common episodes of shellfish contamination by okadaic acid are a human health threat. OA is a secondary metabolite produced by *Dinophysis* and *Prorocentrum* dinoflagellates, easily contaminating various species of shellfish, especially filter-feeding bivalve molluscs, such as mussels, scallops, oysters and clams. Although being not harmful for shellfish, it has some deleterious effects when ingested by humans. OA is one of the major component of the toxic profile associated with diarhetic shellfish poising (DSP). Gastrointestinal symptoms, involving abdominal pain, nausea, vomiting and diarrhoea may occur in the first few hours after consumption of okadaic acid contaminated shellfish, and can remain 3 or 4 days. Contamination of shellfish by okadaic acid occurs in minute quantities and does not confer special taste or odour. It has recently been shown that at infradiarrheic level, OA is cytotoxic and genotoxic, and that its cytotoxicity is enhanced in presence of aluminium or heavy metal.

Okadaic acid is a poly-ether of a C38 fatty acid of formula (I):

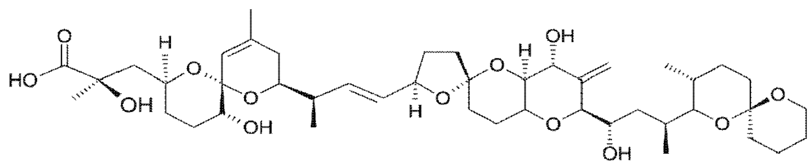

Formula (I)

Other lipophilic toxins derived from OA have a very close formula: dinophysistoxin-1 (DTX1) is identical, except the presence of a supplemental methyl, whereas dinophysistoxin-3 (DTX3) is identical, except the presence of a supplemental acyl group and a supplemental methyl group.

Okadaic acid is also named 9,10-Deepithio-9,10-didehydroacanthifolicin, with a molecular formula: C44H68O13, a molar mass of 805 g mol-1 and IUPAC name: (2*R*)-3-[(2*S*,6*R*,8*S*,11*R*)-2-[(*E*,1*R*)-3-[(2*S*,2&prime*R*,4*R*,4a*S*,6*R*,8a*R*)-4-hydroxy-2-[(1*S*,3*S*)-1-hydroxy-3-[(2*S*,3*R*,6*S*)-3-methyl-1,7-dioxaspiro[5.5]undecan-2-yl]butyl]-3-methylene-spiro[4a,7,8,8a-tetrahydro-4*H*-pyrano[2,3-e]pyran-6,5'-tetrahydrokran]-2'-yl]-1-methyl-prop-2-enyl]-11-hydroxy-4-methyl-1,7-dioxaspiro[5.5]undec-4-en-8-yl]-2-hydroxy-2-methyl-propanoic acid. The mechanism of action of OA is based on the reversible inhibition of protein phosphatase type 2A (PP2A).

Preliminary toxicity screening of lipophilic toxins (okadaic acid, dinophysistoxins, and pectenotoxins) in bivalve molluscs, echinoderms, marine gastropods and tunicates has been traditionally performed using the mouse bioassay, the method of reference recommended by legislation. However, not only the ethical implications but also the poor selectivity and accuracy and the long time required for the analysis, have led the European Commission to recognise alternative methods, such as liquid chromatography coupled to fluorescence (LC-FLD) or mass spectrometry (LC-MS), protein phosphatase inhibition assays or immunoassays (Commission Regulation (EC) No. 2074/2005).

Additionally, several biosensors have been developed for OA assessment, mainly based on the inhibition of protein phosphatase PP2A by OA or on the specific biorecognition using antibodies against OA (Campàs and Marty, 2007; Marquette et al., 1999; Kreuzer et al., 2002; Campàs et al., 2008; Tang et al., 2002; Llamas et al., 2007, Hayat et al., 2012).

EP 0 311 456 discloses a monoclonal antibody to okadaic acids and an assay method for detecting okadaic acid. It discloses the detection by ELISA of okadaic acid previously added at defined concentrations in a sample. EP 0 509 819 discloses an anti-idiotypic monoclonal antibody raised against a mouse anti-OA antibody, said anti-idiotypic antibody being described as an internal image of OA. Detection is made for OA concentration superior to 10 ng/ml. US 5,180,665 discloses a quantitative assay OA based on the ability of OA to specifically inhibit protein phosphatases 1 and 2. This method necessitates fractioning steps prior to detection.

These documents do not disclose an OA specific nucleic acid compound, as does the present invention.

Antibodies have several limitations in regard for use in rapid diagnostic applications. First, they have to be produced in a biological system, which limits the possibility of decreasing the production cost while increasing production amount. The biological production of antibodies also requires a higher level of quality assurance analysis than chemical synthesis. Second, efficient extraction of OA from any sample is performed in the presence of high levels of organic solvents which presence alters the antibody structure and therefore destroys its ligand binding ability. A possible solution

to this problem is to portion a fraction of OA present in organic solvents with an aqueous buffer suitable for use with antibodies, with consequences in terms of amount of OA present in the detection mixture and therefore a loss in sensitivity.

Campas et al (2011) discloses an aptamer-based assay for the detection of okadaic acid. Yang C. et al (2012) discloses aptamer-DNAzyme hairpins for biosensing of Ochratoxin A. The structure of this engineered nucleic acid includes the horseradish peroxidase (HRP)-mimicking DNAzyme and the OTA specific aptamer sequence. These documents do not disclose the sequence of an OA specific nucleic acid compound.

Nevertheless, there is still a lack of highly sensitive and reliable tools able to perform rapid and simple monitoring of real samples to detect and quantify OA.

The inventors have now developed a high affinity ligand for OA and a method of detection of OA using said ligand. The affinity of this isolated nucleic acid compound towards OA allows its use as sensing component of different tools for the development of new assays for determining the presence and/or concentration of OA in samples of interest and/or for removing OA from said sample. The use of a ligand according to the invention as a bio-recognition element in bio-sensing devices provides improved specificity, sensitivity, stability, cost-effectiveness and design flexibility to detection of OA contamination. This is particularly useful for testing food products which are associated with human or animal health risks. The European Commission (Regulation 853/2004) establishes a maximum limit of 160 micrograms of OA-equivalent per kg of bivalve mollusk for OA, dynophysiotoxins and pectenotoxines taken together. The action level established by the FDA is 0,2ppm. A nucleic acid compound according to the invention allows the detection of the analyte at the appropriate regulatory approved levels.

The isolated nucleic acid compounds of the present invention provide significant advantages over prior art methods for the detection of OA in sample material. Indeed, a) they can be chemically synthesized, as the scale of production increases the relative cost per unit of nucleic acid is reduced, b) they can be modified directly through the covalent attachment, in particular to be associated with fluorophores or fluorescence quenching moieties (whereas due to their relatively large mass, antibodies quench fluorescence), this allows a direct measure of the binding interaction between compounds according to the invention and OA, c) they can maintain function within high levels of organic solvent, allowing to perform sample extraction in various conditions, d) they are more thermal stable than antibodies and can be stored for longer periods of time without a noticeable loss of function. Also, nucleic acid compounds according to the invention enable coupling to a large number of surfaces, including a broad selection of resins and glass, therefore allowing further development of various assays. They also allow the detection of the analyte in flow and in batch conditions, and open the possibility for development of integrated automated systems and of high-throughput analysis for OA detection. Finally, their high affinity for OA, with $K_D$ in the nanomolar range, allows the development of high sensitivity assays.

DETAILED DESCRIPTION OF THE INVENTION

The present invention will become more fully understood from the detailed description given herein and from the accompanying drawings, which are given by way of illustration only and do not limit the intended scope of the invention.

The present invention first relates to an isolated nucleic acid compound able to specifically bind to okadaic acid (OA), said nucleic acid compound comprising a nucleotide sequence of at least 30 nucleotides, said nucleotide sequence having at least 80% identity, preferably at least 90% identity, more preferably at least 95% identity and even more preferably at least 98% identity with SEQ ID N°3.

As used herein the terms "Nucleotide sequence" and "sequence" will be employed indifferently in the present specification. The term "isolated nucleic acid" compound means that the nucleic acid compound according to the invention differs from natural nucleic acid compounds. An isolated nucleic acid according to the invention can be prepared by any method known by a person skilled in the art, including chemical synthetic method. A common method of synthesis involves the use of phosphoramidite monomers and the use of tetrazole catalysis. Synthesis of an oligonucleotide starts with the 3' nucleotide and proceeds through the steps of deprotection, coupling, capping, and stabilization, repeated for each nucleotide added.

The term "specific binding" and equivalent phrases refer to the ability of a binding moiety, being for example an isolated nucleic acid or okadaic acid, to bind preferentially to a particular target molecule, for example okadaic acid or an isolated nucleic acid, in the presence of a heterogeneous population of proteins and other components, without significant binding to other components present in a test sample. Typically, specific binding between two entities means a binding affinity of at least about $10^{-6}$ M, and preferably at least about $10^{-7}$M, $10^{-8}$M, $10^{-9}$M or $10^{-10}$M. Specific (or selective) binding can be assayed and specific binding molecules identified by any method known by a person skilled in the art. Said methods are well known in the art and include, for example, equilibrium dialysis, surface plasmon resonance, and the like, as described in the following Examples.

Typically a specific, or selective, reaction according to this assay is at least about twice background signal or noise and more typically at least about 5 or at least about 100 times background. By "specifically binding", "specifically binds", "recognizing" or the like, it is also intended herein that, the isolated nucleic acid according to the invention forms

with OA a complex that is relatively stable under physiological conditions and in assay conditions.

In a population of candidate nucleic acids, a nucleic acid ligand according to the invention is one which binds to OA with greater affinity than that of the bulk population. The term "nucleic acid ligand" herein refers to an isolated nucleic acid compound able to specifically bind to okadaic acid according to the invention.

Kinetic-based equilibrium dissociation constant ($K_D$) is determined as a key parameter to evaluate strength of the binding of a molecule for another molecule. Typically, a smaller $K_D$ means a greater affinity. The specific binding of an isolated nucleic acid according to the invention for OA is characterized by a $K_D$ comprised between about $10^{-7}$M and about $10^{-11}$M, preferably of about $10^{-8}$M to about $10^{-10}$M, and more specifically of about $10^{-9}$M, wherein the term "about" indicates a possible variation of +/-10%.

According to the invention, an isolated nucleic acid compound able to specifically bind to okadaic acid is also able to bind to toxins often described as "okadaic acid derivatives", said "okadaic acid derivatives" being chosen among the group consisting of: okadaic acid, dinophysistoxin-1 (DTX-1), dinophysistoxin-3 (DTX-3) and pectenotoxins.

In a particular embodiment, the present invention relates to an isolated nucleic acid compound able to bind specifically to okadaic acid, said nucleic acid comprising a nucleotide sequence of at least 30 nucleotides having at least 80% identity with the nucleotide sequence SEQ ID N°3. In another particular embodiment, the invention relates to an isolated nucleic acid sequence wherein said sequence of nucleotides comprises at least 40 nucleotides, preferably at least 50 nucleotides, and more preferably at least 60 nucleotides. In a particular embodiment, the invention relates to an isolated nucleic acid sequence comprising 62, 61 or 60 nucleotides. The term "sequence identity" herein means that two nucleotide sequences are identical (i.e. on a nucleotide-by-nucleotide basis) over a window of comparison. The "percentage of sequence identity" is calculated by comparing two optimally aligned sequences over the window of comparison, determining the number of positions at which the identical residues occurs in both sequences to yield the number of matched positions, dividing the number of matched positions by the total number of positions in the window of comparison (i.e., the window size), and multiplying the result by 100 to yield the percentage of sequence identity. The percentage of sequence identity of a nucleotide sequence can also be calculated using BLAST software with the default or user defined parameter.

SEQ ID N°3 comprises a sequence of 63 nucleotides. To develop improved nucleic acid ligands for OA, a person skilled in the art may design and synthesize shorter oligonucleotides based on the sequence SEQ ID N° 3. In a particular embodiment, shorter nucleotides based on the sequence SEQ ID N°3 may comprise 62, 61 or 60 nucleotides. A reduction in oligonucleotide size allows a cost saving for synthesis of the molecule.

In a particular embodiment, an isolated nucleic acid compound according to the present invention comprises a sequence having at least 90% identity with the nucleotide sequence SEQ ID N°3. In a more particular embodiment, said compound according to the present invention comprises a sequence having at least 95% identity with the nucleotide sequence SEQ ID N°3. In an even more particular embodiment, said compound according to the present invention comprises a sequence having at least 98% identity with the nucleotide sequence SEQ ID N°3. In an even more particular embodiment, an isolated nucleic acid compound according to the present invention comprises a sequence having 100% identity with the nucleotide sequence SEQ ID N°3. In an even more particular embodiment, the invention relates to an isolated nucleic acid compound whose nucleotide sequence is SEQ ID N°3.

In another particular embodiment, the present invention also relates to an isolated nucleic acid compound able to bind specifically to okadaic acid, said sequence comprising a nucleotide sequence of at least 30 nucleotides and having at least 80% identity with a nucleotide sequence chosen among the group consisting of: SEQ ID N°1, SEQ ID N°2, SEQ ID N°4, SEQ ID N°5, SEQ ID N°6, SEQ ID N°7 and SEQ ID N°8. In a particular embodiment, an isolated nucleic acid compound according to the present invention comprises a sequence having at least 90% identity with a nucleotide sequence chosen among the group consisting of: SEQ ID N°1, SEQ ID N°2, SEQ ID N°4, SEQ ID N°5, SEQ ID N°6, SEQ ID N°7 and SEQ ID N°8. In an even more particular embodiment, an isolated nucleic acid compound according to the present invention comprises a sequence having 100% identity with a nucleotide sequence chosen among the group consisting of: SEQ ID N°1, SEQ ID N°2, SEQ ID N°4, SEQ ID N°5, SEQ ID N°6, SEQ ID N°7 and SEQ ID N°8. In an even more particular embodiment, an isolated nucleic acid compound according to the present invention has a sequence having 100% identity with a nucleotide sequence chosen among the group consisting of: SEQ ID N°1, SEQ ID N°2, SEQ ID N°4, SEQ ID N°5, SEQ ID N°6, SEQ ID N°7 and SEQ ID N°8.

In another particular embodiment, an isolated nucleic acid compound able to bind to okadaic acid, according to the present invention, is an aptamer. Aptamers are alternative biorecognition elements introduced for the first time in the early 1990s. The term aptamer comes from the Latin word "aptus", meaning "fitting", and the Greek word "meros", meaning "particle". Aptamers are small single-stranded DNA or RNA molecules (40-100 bases) selected from a random oligonucleotide library to bind a target molecule. Their three-dimensional shape is responsible for their high affinity and selectivity. They can discriminate between closely related compounds on the basis of subtle structural differences. These synthetic affinity probes can be developed for almost any target molecule, covering a wide range of applications in fields such as environmental monitoring, food control, clinical diagnosis and therapeutics.

The inventors report here the identification and characterisation of single-stranded DNA aptamer to the phycotoxin OA that was isolated from a 63-nucleotides random library with a theoretical diversity of $10^{13}$ to $10^{15}$ different species. The aptamer selection process, termed SELEX (Systematic Evolution of Ligands by EXponential enrichment) is an iterative process based on *in vitro* selection and enzymatic amplification techniques that mimics a Darwinian-type selection of relatively few, but optimised structural motifs (Ellington and Szostak, 1990; Tuerk and Gold, 1990; Stoltenburg et al., 2007). Fluorescent labels have been used for DNA quantification and magnetic beads for target immobilisation. Immobilization on magnetic beads enabled easy handling, use of very small amounts of target for the aptamer selection, rapid and efficient separation of bound and unbound molecules, and stringent washing steps. Stoltenburg and co-workers (2005) called this modified SELEX technology FluMag-SELEX.

In a particular embodiment, the present invention relates to an isolated nucleic acid compound able to bind to okadaic acid which is a DNA molecule. In a more particular embodiment, the present invention relates to an isolated nucleic acid compound which comprises a "functionnally equivalent variant", a "functional analogue" or a "functional fragment" of the nucleotide sequence SEQ ID N°3. In another particular embodiment, an isolated nucleic acid compound able to bind to okadaic acid, according to the present invention, comprises a "functionnally equivalent variant", a "functional analogue" or a "functional fragment" of a nucleotide sequence chosen among the group consisting of: SEQ ID N°1, SEQ ID N°2, SEQ ID N°4, SEQ ID N°5, SEQ ID N°6, SEQ ID N°7 and SEQ ID N°8. This includes, but is not limited to, nucleotides with partial sequence homology, nucleotides having one or more specific conservative and/or non-conservative base changes which do not alter the biological or structural properties of the ligand according to the invention, said functional property being the specific binding of okadaic acid.

A plurality of distinct oligonucleotides with different substitutions may be made, with such molecules exhibiting an equivalent functional biological activity. It may be well understood by persons skilled in the art that in the definition of a biologically functional molecule there is a limit to the number of changes that may be made within a defined portion of the molecule. The DNA ligand analogues of the instant invention also encompass oligonucleotides that have been modified by the inclusion of non-natural nucleotides including but not limited to, 2,6-Diaminopurine-2'-deoxyriboside, 2-Aminopurine-2'-deoxyriboside, 6-Thio-2'-deoxyguanosine, 7-Deaza-2'-deoxyadenosine, 7-Deaza-2'-deoxyguanosine, 7-Deaza-8-aza-2'-deoxyadenosine, 8-Amino-2'-deoxyadenosine, 8-Amino-2'-deoxyguanosine, 8-Bromo-2'-deoxyadenosine, 8-Bromo-2'-deoxyguanosine, 8-Oxo-2'-deoxyadenosine, 8-Oxo-2'-deoxyguanosine, Etheno-2'-deoxyadenosine, N6-Methyl-2'-deoxyadenosine, O6-Methyl-2'-deoxyguanosine, 06-Phenyl-2'-deoxyinosine, 2'-Deoxypseudouridine, 2'-Deoxyuridine, 2,4-Difluorotoluyl, 2-Thiothymidine, 4-Thio-2'-deoxyuridine, 4-Thiothymidine, 5'-Aminothymidine, 5'-Iodothymidine, 5'-O-Methylthymidine, 5,6-Dihydro-2'-deoxyuridine, 5,6-Dihydrothymidine, 5-(C2-EDTA)-2'-deoxyuridine, 5-(Carboxy)vinyl-2'-deoxyuridine, 5-Bromo-2'-deoxycytidine, 5-Bromo-2'-deoxyuridine, 5-Fluoro-2'-deoxyuridine, 5-Hydroxy-2'-deoxycytidine, 5-Hydroxy-2'-deoxyuridine, 5-Hydroxymethyl-2'-deoxyuridine, 5-Iodo-2'-deoxycytidine, 5-Iodo-2'-deoxyuridine, 5-Methyl-2'-deoxycytidine, 5-Propynyl-2'-deoxycytidine, 5-Propynyl-2'-deoxyuridine, 6-O-(TMP)-5-F-2'-deoxyuridine, C4-(1,2,4-Triazol-1-yl)-2'-deoxyuridine, N4-Ethyl-2'-deoxycytidine, O4-Methylthymidine, Pyrrolo-2'-deoxycytidine, and Thymidine Glycol.

In a more specific embodiment, the present invention relates to an isolated nucleic compound according to the present invention which $K_D$ is comprised between $10^{-8}$M (+/- 10%) and $10^{-10}$M (+/- 10%), and more specifically which is of $10^{-9}$M (+/-10%).

In a particular embodiment, the invention relates to an isolated nucleic acid compound chemically coupled to a labeling element. As used herein, a labeling element according to the invention is an element allowing the detection of the presence of the nucleic acid compound of the invention. In a particular embodiment, said labeling element allows an indirect detection and is, for example but without limitation, an enzyme which substrate is detectable. In another particular embodiment, said labeling element allows a direct detection and is, for example but without limitation, a fluorophore.

In a more particular embodiment, the invention relates to a nucleic acid compound chemically coupled to an enzyme. In another particular embodiment, the present invention relates to a nucleic acid compound chemically coupled to a DNAzyme hairpin, wherein the structure of this engineered nucleic acid includes an enzyme mimicking DNAzyme and a nucleic acid compound able to bind to OA. More particularly, the present invention relates to an aptamer coupled to DNAzyme hairpin including an aptamer able to bind to OA. As an example, the present invention relates to the horseradish peroxidase (HRP)-mimicking DNAzyme coupled to an OA binding aptamer (Yang et al, 2012). Examples of the enzyme used in the labelling include in particular peroxidase, including horseradish-peroxidase (HRP), alkaline phosphatase (ALP), beta-galactosidase, catalase, glucose oxidase, lactic acid oxidase, alcohol oxidase and monoamine oxidase. They may be used singly or in combination.

In another particular embodiment, the invention relates to a nucleic acid compound chemically coupled to labeling element allowing the direct detection of the compound. In a particular embodiment, said labeling element can be a magnetic particle, or an equivalent thereof. The present invention relates in particular to a nucleic acid compound chemically coupled to a marker chosen among the following: a fluorophore, a biotin molecule, a streptavidin molecule. By fluorophore, it is herein meant any fluorescent molecule, i.e. a molecule capable of re-emitting light upon light excitation, or other electromagnetic light. The fluorophore is for example chosen from Xanthene, Cyanine, Naphthalene, Coumarin,

Oxadiazole, Pyrene, Oxazine, Acridine, Arylmethine or Tetrapyrrole derivatives. Labeling of an isolated nucleic acid according to the invention may be carried out by a method known by a person skilled in the art.

According to a particular embodiment, the present invention relates to a method for detecting okadaic acid in a sample, said method comprising the steps of: (a) providing a biological sample, (b) contacting said sample to an isolated nucleic acid according to the invention under conditions wherein an okadaic acid/nucleic acid complex may be formed; and (c) using detection means to determine whether said okadaic acid/nucleic acid complex is formed, thereby determining the presence of okadaic acid in said sample.

According to another embodiment, the present invention relates to a method for determining the concentration of okadaic acid in a sample comprising the steps of: (a) providing a sample, (b) contacting said sample with an isolated nucleic acid according to the invention under conditions wherein an okadaic acid/nucleic acid complex may be formed; and (c) quantitatively detecting the okadaic acid/nucleic acid complex, thereby determining the concentration of the okadaic acid in the sample.

The method is based on specific recognition of OA by isolated nucleic acids of the invention. According to a further aspect, a method of the present invention comprises the steps of: (a) reacting known concentrations of OA with a nucleic acid according to the invention, under conditions wherein an OA/nucleic acid complex may be formed, to construct a calibration curve representing the relation between the detected signal of the OA/nucleic acid complex and the concentration of OA; (b) providing a biological sample, (c) contacting said biological sample with an isolated nucleic acid according to the invention under conditions wherein an okadaic acid/nucleic acid complex may be formed; and (d) quantitatively detecting the okadaic acid/nucleic acid complex, thereby determining the concentration of the okadaic acid in the sample, by interpolating the data obtained from the standard curve.

According to another embodiment, the present invention relates to methods using a nucleic acid compound of the invention, including a method for removing or reducing the level of okadaic acid from a biological sample, a method for purifying okadaic acid from a biological sample, a method for modifying the biological function of OA, said method comprising the inhibition and/or neutralization of the biological function of OA.

According to a particular embodiment, the present invention relates to a method for detecting okadaic acid and/or to a method for determining the concentration of okadaic acid in a biological sample. A biological sample that can be assayed with a method according to the present invention may be for example a water sample or a marine sample. Said marine sample may be for example a preparation comprising phytoplancton, or the like, or a preparation obtained from shellfish, such as a shellfish extract. In a particular embodiment, the invention relates to a method wherein said biological sample is obtained from shellfish, preferably from a filter-feeding bivalve mollusc, and more particularly from a bivalve mollusc selected among the group consisting of: oysters, mussels, scallops and clams.

The preparation of a biological sample from seafood for OA detection and/or for OA quantitation is performed according to any method known by a person skilled in the art. As an example, mussels or oysters are removed from their shells, washed with deionized water, thoroughly dried and homogenized. In the case of the presence of dyes, such as in mussel extract, a purification step may be performed. A sample pretreatment step could be a purification step, such as chromatography, dialysis or a method known by a person skilled in the art.

Another embodiment of this invention would include a sample pretreatment step to reduce the concentration of potential contaminating compounds. In one aspect, the extract of the sample is an organic solvent extract of the sample. Organic solvents that may be used in accordance with the present invention include, but are not limited to, methanol, ethanol and acetonitrile.

In a particular embodiment, a method according to the invention comprises OA detection under equilibrium conditions; in another particular embodiment a method according to the invention comprises OA detection via real-time monitoring.

In a particular embodiment of the invention, the method comprises the immobilization on a solid phase support of a ligand; in a particular embodiment, said ligand is OA; in another particular embodiment said ligand is a nucleic acid according to the invention, able to bind to OA. Said solid-phase support may be, for example:

- plates, including an immunoplate, such as a 96-well microplate,
- beads, such as polyethylene beads, polystyrene beads, ABS resin beads, magnetic beads
- metallic nanoparticles, such as gold or silver nanoparticles, non-metallic nanoparticles
- solid phase of a chromatography column, such as affinity column,
- electrodes, including carbon or gold electrodes
- membranes, such as nitrocellulose membranes

A ligand may be fixed on a solid support by physical adsorption, chemical coupling or any method known by a person skilled in the art. Said immobilization may be performed via direct coupling or by indirect coupling using a spacer. In a more particular embodiment, OA is fixed to a solid-phase support, such as plates or beads, more particularly to an immunoplate, such as a 96-well microplate, polyethylene beads, polystyrene beads or ABS resin beads. More specifically,

an OA-high molecular weight protein complex is contacted with a solid-phase support and fixed. As an example, OA might be complexed, or chemically coupled, to a high molecular weight protein, such as bovine serum albumin (BSA) or biotin.

In another particular embodiment, an isolated nucleic acid able to bind specifically to OA is fixed to a solid-phase support, such as plates or beads, more particularly to an immunoplate, such as a 96-well microplate, polyethylene beads, polystyrene beads or ABS resin beads.

In a particular embodiment, a method according to the invention comprises the indirect detection of OA, or determination of the concentration of OA via a competitive assay. In another particular embodiment, a method according to the invention comprises the direct detection of OA, or determination of OA concentration.

In an indirect detection assay, the assay reagent specific for OA competitively reacts with OA fixed on the support and with OA possibly present in the tested sample. As a result, the amount of okadaic acid in the sample can be measured. In a particular embodiment, the invention relates to a method comprising a competitive assay chosen among the following: enzyme-linked oligonucleotide assay (ELONA) and kinetic exclusion assay (KinExA).

In a particular embodiment, the invention relates to a method wherein the formation of the okadaic acid/nucleic acid complex is detected by a method chosen among the following: visual detection, optical density detection, fluorescence detection, colorimetric detection, radioactivity detection, electrochemical detection using devices such as amperometric, impedimetric or FET-devices, luminescent detection such as electrochemiluminescent, quartz-crystal microbalance or surface enhanced Raman spectroscopy-based methods. Also, a method for the detection of the formation of the okadaic acid/nucleic acid complex may be a chromatographic strip assays based on visual detection.

In a direct detection assay, the nucleic acid compound according to the invention is contacted with a support and fixed to it. Said compound directly reacts with OA possibly present in the tested sample. As a result, the amount of okadaic acid in the sample can be measured. In a particular embodiment, the invention relates to a method comprising an assay performed by Surface Plasmon Resonance (SPR).

In another particular embodiment, the invention relates to a method wherein the contacting step is performed in the presence of an organic solvent chosen among the group consisting of: methanol, dimethyl sulfoxide, ethanol, and acetonitrile. Approved methods for the extraction of OA from biological samples all involve the use of organic solvents such as methanol or acetonitrile. Dimethyl sulfoxide (DMSO) is also frequently used to accelerate solubility of OA. The inventors of this invention realized that there was a need for a detection system that would tolerate higher levels of organic solvents such as these for use in the detection of OA. In one aspect of the invention the extract is an organic solvent extract of the sample. In another aspect the organic extract solution may be diluted to a level where the organic solvent is tolerated by the nucleic acid compound of the invention, which can be, for example, 5% to 25% methanol, or 10% ethanol.

In another particular embodiment, the invention relates to a method wherein the contacting step is performed at a pH value comprised between about pH 4 and about pH 5, the optimal pH preferably of about pH 4,5 wherein the term "about" indicates a possible variation of +/-10%.

The optimum pH range for detection being comprised between about pH 4 and about pH 5, the addition of a buffer to adjust the matrix's pH might be necessary.

According to a further aspect of the present invention, a composition comprising a cation for enhancing the affinity of a nucleic acid ligand to a target is provided, wherein said cation is selected from the group consisting of: magnesium, sodium, potassium, calcium, scandium, titanium, vanadium, chromium, manganese, iron, cobalt, nickel, copper and zinc. In one aspect of the present invention, a composition comprising a calcium cation for enhancing the affinity of a nucleic acid ligand to a target is provided.

Fluorescence-based means may be used to detect the presence of OA and for measuring the concentration of OA in samples. Other detection means that can be used in aspects of the present invention include, without limitation, the use of fluorescence, or fluorescence in combination with quenchers, or fluorescence polarization, and electro-affinity analysis of OA/nucleic acid complex formation. In one aspect of the present invention, the concentration of OA may be determined on the basis of a spectral shift in the fluorescence spectrum of OA due to the binding of OA to the nucleic acid compound of the invention.

In another particular embodiment, the present invention also relates to kits comprising isolated nucleic acid binding to OA according to the invention. In a more particular embodiment, the present invention also relates to kits for determining the amount of okadaic acid and derivatives thereof in a sample. In a more particular embodiment, isolated nucleic acid binding to OA in a kit according to the invention is present in a soluble form. In a more particular embodiment, the nucleic acid is associated with a label allowing its detection. In a particular embodiment, said nucleic acid is covalently bound to a detectable label being, for example a fluorophore or an enzyme. More particularly, said nucleic acid according to the invention which has been coupled to fluorescein or an enzyme such as alkaline phosphatase or HRP.

In a first embodiment, a kit according to the present invention comprises isolated nucleic acid binding to OA according to the invention and at least a solid surface having been coated with or bound to OA. Said solid surface coated with OA may be, for example, a microtiter plate or beads. Different OA-conjugates may be immobilized on said solid

surfaces, they may be, for example, OA-BSA or OA-biotine, or the like. In another particular embodiment, the present invention also relates to kits comprising isolated nucleic acid binding to OA according to the invention, solid surface having been coated with OA and reagents for the detection of the presence of an okadaic acid/nucleic acid complex. In a more particular embodiment, a kit according to the present invention comprises at least a solid surface having been coated with or bound to OA, a solution comprising isolated nucleic acid according to the invention and solution comprising agents allowing the detection of the presence of the isolated nucleic acid according to the invention.

In a particular embodiment, the invention relates to a kit comprising at least a microtiter plate coated with OA, standard solutions comprising defined concentrations of OA, a solution comprising a nucleic acid according to the invention covalently associated with a fluorophore, and auxiliary solutions and reagents. In a second embodiment, a kit according to the present invention comprises at least a solid surface having been coated with or bound to an isolated nucleic acid according to the invention. Said solid surface may be, for example, a microtiter plate, beads, or a sensor chip. In a particular embodiment, said sensor chip is adapted for Surface Plasmon Resonance and is, for example, a modified CM5 gold chip bearing adapted oligonucleotides.

According to a further aspect of the present invention, a composition comprising a cation for enhancing the affinity of a nucleic acid ligand to a target is provided.

In another embodiment, the present invention relates to a device comprising at least a nucleic acid compound according to the invention, said nucleic acid compound having been immobilized on a solid phase support.

In another embodiment, the present invention relates to the use of an isolated nucleic acid according to the invention used for the detection of OA. The novel OA-binding nucleic acids of the present invention may be involved in a variety of uses and applications characterized by the binding of the nucleic acid ligands of the present invention to OA. Said use can be any one of the following: detecting okadaic acid in a biological sample, determining the concentration of okadaic acid in a biological sample, removing or reducing the level of okadaic acid from a biological sample, purifying okadaic acid from a biological sample, modifying the biological function of OA.

LEGENDS OF THE FIGURES

Figure 1: Scheme of the SELEX process used to select a DNA aptamer for OA.

Figure 2: Calibration curves resulting from the non-linear four-parameter logistic regression fitting of the plots obtained for different aptamer concentrations when fixing the OA-BSA coating concentration for each curve. The detected absorbance at 620 nm is represented as a function of the aptamer concentration, in nM.

Figure 3: Globally fit Biacore data of aptamer binding to immobilized OA. The graph represents the intensity of the response as a function of time. Eleven different concentrations of aptamer were injected in duplicate from 1nM to 100nM. The amplitude of the response is represented as a function of time.

**Figures 4A and 4B**: Colorimetric assay based on an indirect competitive approach using OA-modified magnetic beads. Figure 4A: Scheme of the colorimetric assay.

Figure 4B: calibration curve, error bars are standard deviations of the mean with n=3.

The percentage of competition is represented as a function of OA concentration, in nM.

**Figures 5A and 5B:** KinExA-based aptasensing system used to detect OA, using OA-BSA- and biotinylated OA-modified PMMA beads. Figure 5A: Scheme of the KinExA system. Figure 5B: Calibration curves obtained using OA-BSA- and biotinylated OA-modified PMMA beads, error bars are standard deviations of the mean with n=3. The percentage of fluorescence is represented as a function of aptamer concentration, in nM.

**Figures 6A and 6B**: SPR assay based on the direct detection of OA onto aptamer-modified gold chips. Figure 6A: Scheme of the SPR assay based on the direct detection of OA onto aptamer-modified gold chips. Figure 6B: Calibration curves obtained using unlabelled aptamer, biotinylated aptamer and ALP-aptamer-modified surfaces. RU is represented as a function of OA concentration, in nM.

EXAMPLES

**Example 1: Selection of OA binding DNA aptamers**

*Reagents and materials*

A ssDNA library (Tsukuba oligo service, Japan) with a theoretical diversity of $10^{15}$ was used as initial oligonucleotide pool for the aptamer selection process. The 63-nucleotides random sequence was flanked by 18-nucleotides primers on either side. 5'-GTACCAGCTTATTCAATT-3' or 5'-fluorescein-C6-GTACCAGCTTATTCAATT-3' were used as forward primer, and 5'-biotin-C6-CTGATGAACATACTATCT-3' was used as reverse primer. These DNA molecules

would be considered by one trained in the art as essentially random, as they were not selected following exposure to OA. Primers, dNTP and *Ex Taq* (*Taq* DNA polymerase) were from TaKaRa (Japan). Biotinylated aptamer was synthesized by Eurogentec (Belgium). Amine-modified magnetic beads (Dynabeads M-270 Amine) were supplied by Invitrogen Japan K.K. Neutravidin-agarose was acquired from Funakoshi (Japan). OA potassium salt was purchased from Wako Pure Chemical Industries (Osaka, Japan). OA standard solutions were firstly prepared in ethanol ($0.1 g•L^{-1}$) and subsequently diluted in the proper buffered solution. Bovine serum albumin (BSA) and 3,3',5,5'-tetramethylbenzidine (TMB) liquid substrate were purchased from Sigma Chemical (St. Louis, MO, USA). HRP-Streptavidin conjugate was purchased from Fischer Scientific (France). EZ-Link amine-$PEO_3$-biotin, avidin, hydroxylamine, *N*-hydroxysuccinimide (NHS), *N*-(3-dimethylaminopropyl)-*N*'-ethyl-carbodiimide hydrochloride (EDC) and components of buffers were also supplied by Wako Pure Chemical Industries, Ltd. (Osaka, Japan). All solutions were prepared using Milli-Q water. 96-Well Maxisorp microtiter plates were obtained from Nunc™ (Denmark). SPR sensor chips based on a carboxymethylated dextran matrix covalently attached to the gold surface (CM5) were purchased from GE Healthcare. Poly(methyl methacrylate) (PMMA) beads (100-μm diameter) were supplied by Sapidyne Instruments (Boise, ID, USA).

Colorimetric measurements were performed with a Lab-systems Multiskan EX microtiter plate reader (Thermo Life Sciences, Cergy-Pontoise, France). SPR measurements were performed with a Biacore T100 system (GE Healthcare). A KinExA 3000 instrument (Sapidyne Instruments, Boise, ID, USA) was used as immunoassay platform for kinetic exclusion measurements.

OA was conjugated to BSA using EDC, slightly modifying the procedure previously described by Levine et al. (1988) and Tang et al. (2002). After reaction, extensive dialysis was performed first with 0.05M carbonate buffer, to remove any uncoupled OA, and then with milliQ water to remove carbonate salts, using a 10,000 MWCO dialysis cassette (Slide-A-Lyzer, Pierce). OA was conjugated to biotin according to the procedure previously described (Prieto-Simón et al., 2010). Briefly, biotinylated OA was synthesised using a commercial carboxyl reactive biotin labelling reagent (EZ-Link amine-PEO3-biotin), slightly modifying the procedure detailed by Pierce. After biotinylation, the reaction mixture was transferred into a dialysis device (Spectra/Por Float-A-Lyzer), fitted with a 1,000 MWCO cellulose ester membrane (Spectrum Laboratories Inc., CA, USA), and dialysed several times against 0.05M carbonate buffer and finally against milliQ water. Aliquots of both conjugates, OA-BSA and OA-biotin, were stored at 4°C for daily use. Carboxyl-group of OA and amine-groups on the surface of the amine-modified magnetic beads were linked together by using EDC/NHS in 0.1M MES buffer, pH 5.5. Following the procedure detailed by Dynal Biotech, 180μg OA were used to react with 75μL of beads to obtain a final concentration of $1x10^9$ beads per mL. Similarly, magnetic beads were modified with acetic acid to be used as control beads for counter selection steps. Extensive washing of the coated beads was required to remove excess ligand.

PMMA beads were coated with OA-BSA conjugate or biotinylated OA depending on the strategy. Approximately 220mg 100μm diameter PMMA beads were suspended in 1mL of PBS containing 23μg of OA-BSA conjugate and rotated overnight. After settling and removal of the supernatant solution, 1mL of $10g•L^{-1}$ BSA solution in PBS was added as blocking agent to minimise non-specific adsorptions, and the beads were rotated for 1h. For biotinylated OA-modified beads, firstly beads were suspended in 1mL PBS containing 0.5mg of avidin and rotated overnight. Avidin-modified beads were then resuspended in 1mL of PBS containing 2μg of biotinylated OA and rotated for 4h. After removal of supernatant, blocking with a $10g•L^{-1}$ BSA solution was performed. Coated beads were stored at 4°C and diluted to 27mL with PBS prior to use.

*Selection process*

Aptamers that bind to OA were selected using a modified SELEX technology called FluMag-SELEX, based on fluorescent labelled primers for DNA quantification and magnetic beads for OA immobilisation. The selection process was based on the repetition of five main steps: binding, partition, elution, amplification and conditioning (Figure 1). Prior to the first round of selection, 3nmol of a 99-mer single strand DNA (ssDNA) with a random insert of 63 nucleotides were denaturated in a selection buffer (10mM $NaHPO_4$, 2mM $KH_2PO_4$, 138mM NaCl, 2.7mM KCl, 2.5mM $MgCl_2$ and 0.05% Tween, pH 7.4) at 95°C for 5 min and then allowed to cool to room temperature for 30min. Binding step takes place when this denatured ssDNA random library is exposed to 10μL of OA-modified magnetic beads (approximately $1x10^7$ beads). After 30min incubation at room temperature, the oligonucleotides bound to OA-modified beads were partitioned from unbound and weakly bound oligonucleotides using a magnet. Extensive wash of the beads was performed with the same selection buffer. This crucial step strongly affects the success of the aptamer selection. Bound oligonucleotides were eluted by incubating the beads in elution buffer (40mM Tris-HCl, 10mM EDTA, 3.5M urea and 0.02% Tween, pH 8.0) at 80°C during 7min. This elution step was repeated and the collected ssDNA was purified by ethanol precipitation in the presence of glycogen. This ssDNA was amplified by Polymerase Chain Reaction (PCR), using a fluorescein-modified forward primer and a biotinylated reverse primer. The thermal cycling was 94°C for 30s, 46°C for 30s and 72°C for 30s. After ethanol precipitation of the PCR products, they were passed through a neutravidin-agarose column to obtain an enriched ssDNA pool of selected oligonucleotides ready for the next round of selection. The enriched library

obtained after every three cycles was incubated with acetic acid-modified magnetic beads as negative selection to remove the ssDNA which had bound to the free binding sites on the magnetic beads. Iterative cycles of selection and amplification resulted in an enrichment of relatively few sequence motifs showing the highest affinity and specificity towards OA. After the amplification step of the last round, the SELEX process was stopped and the PCR products were cloned in plasmids (pGEM-T vector, Promega) into bacteria to obtain individual aptamer clones from the selected pool. Finally, these individual aptamers were sequenced by the dideoxy method.

*Results*

The nucleotide sequence corresponding to the random region of the 8 selected aptamers, namely aptamers 1, 2, 3, 7, 8 10, 11 and 12, are respectively described as SEQ ID N°1, SEQ ID N°2, SEQ ID N°3, SEQ ID N°4, SEQ ID N°5, SEQ ID N°6, SEQ ID N°7 and SEQ ID N°8. Aptamer 3 (SEQ ID N°3) was chosen for further characterisation.

**Example 2: Characterization of OA binding aptamer by ELONA**

An Enzyme-Linked OligoNucleotide Assay (ELONA) protocol was developed for functional affinity determination of the selected aptamer on the basis of a primary coating of 96-well microtiter plates with OA (Figure 4A). Colorimetric checkerboards were performed in order to optimise the protocol, minimising diffusion effects and reaching the state of equilibrium necessary for heterogeneous solid-phase affinity measurements (Loomans et al., 1995). The first step was the adsorption of the OA-BSA conjugate onto microtiter wells using four different OA-BSA-coating concentrations: 3.9nM, 7.8nM, 15.6nM and 31.2nM (coating concentrations ratio 1:2:4:8). Decrease of $OD_{100}$ values for two consecutive sigmoid curves must be between 35 and 65%. Each OA-BSA concentration was prepared in 0.05M carbonate buffer, pH 9.6. The microtiter plates were incubated overnight at 4°C. After blocking the wells with 3% BSA solution in PBS, 1-h incubation step was performed with different biotinylated aptamer/streptavidin-HRP solutions, based on a protocol such as described in Barthelmebs et al. (2011). The biotinylated aptamer having a nucleotidic sequence SEQ ID N°3 was synthesized by Eurogentec (Belgium). Different concentrations of biotinylated aptamer were used, from 5pM to 10nM, while the concentration of streptavidin-HRP was fixed at $0.1 mg{\bullet}L^{-1}$. Finally, 100μL of HRP substrate solution (TMB liquid substrate) was incubated for the colour development step. The reaction was stopped after appropriate time. Absorbance values were measured at 620nm. Each step was performed at room temperature with shaking and protected from light. The wells were thoroughly rinsed three times with 0.1M PBS containing 0.05% Tween 20 (PBS-T) between each step. Assays were performed in triplicate. The same protocol could be performed with aptamers having a nucleotidic sequence chosen among the group consisting of SEQ ID N°1 to SEQ ID N°8.

*Results and conclusion*

OA-BSA coating concentrations were taken from the linear part of the sigmoidal dose response curve using an excess of aptamer, to set a non-competitive coating. The four OA-BSA concentrations, following the ratio 1:2:4:8, were chosen to provide a decrease of $OD_{100}$ values between 35 and 65% for two consecutive sigmoidal curves. Under these optimised conditions, determination of the functional affinity constant was appropriate based on the application of the Law of Mass Action and hence of the Beatty formula (Loomans et al., 1995). Data shown in Figure 2 was used to calculate the functional affinity constant for the OA aptamer, where n = [OA]/[OA]', with:

$$K_{aff} = [\text{OA-aptamer}] / [\text{OA}] [\text{aptamer}] = (n-1) / 2(n[\text{aptamer}]' - [\text{aptamer}]).$$

The selected aptamer showed a $K_{aff}$ of $0.19 \times 10^9 M^{-1}$ and thus a $K_D$ of 5.4nM. The obtained $K_D$ value in the nM range is the result of a reasonably stable three-dimensional structure of the aptamer that makes more difficult the spontaneous unfolding and subsequent release of the OA.

**Example 3: Characterization of OA binding aptamer by Kinetic Exclusion Assay**

*Reagents and material*

A KinExA 3000 instrument (Sapidyne Instruments, Boise, ID, USA) was used as aptamer-based assay platform for kinetic exclusion measurements (Drake et al., 2004). Two different protocols were followed, depending on the OA conjugate immobilised on the PMMA beads: OA-BSA conjugate or biotinylated OA (Figure 5A). Details of the KinExA instrument performance have been described elsewhere (Blake et al., 1999; Khosraviani et al., 2000). Briefly, the automated flow assay system consists of a capillary flow cell fitted with a microporous screen where beads are packed acting as the solid phase. Solutions are drawn through the flow cell due to the negative pressure created by a syringe pump. All KinExA experiments were conducted at room temperature. Initially, preliminary measurements were performed in order to choose aptamer concentration. The single strand DNA sequence was amplified using the fluorescent forward primer (5'-fluorescein-C6-GTACCAGCTTATTCAATT-3'). FITC-aptamer was obtained after PCR amplification and purification using a neutravidin-agarose column. Different fluorescein-modified aptamer concentrations (from 10pM to 100nM) were drawn through the OA-modified beads to accumulate unbound aptamer. The fluorescein label enabled the fluorescence detection. Even when using very low concentrations of aptamer, the measured signal was enough due to the accumulation effect enabled by the continuous flow. In order to ensure that non-specific adsorption took place, an additional solution of fluorescein-labelled primer (P18) was also injected, using concentrations in excess compared to those used for the aptamer for each assay (150nM labelled-primer for OA-BSA-modified beads and 73.5nM labelled-primer for avidin-biotinylated OA-modified beads). Equilibrium experiments were performed with OA serially diluted into solutions having a constant aptamer concentration. OA/aptamer mixtures were allowed to reach equilibrium by incubating overnight at room temperature. Pumps and valves to perform all the steps were controlled by a computer system. Optimisation of volumes and concentrations of reagents, as well as of flow rates and times of reagent handling through the microfluidic system was performed. Data were fit to a 1:1 binding model using KinExA software.

*Results and conclusion*

Optimisation studies of sample volume, flow rate, injection time and aptamer concentration, were performed in order to obtain approximately a signal of 1.0V for the relative signal difference (RSD), being the difference between the signal 100% (signal produced when 100% of the binding sites are free at a given concentration) and the non-specific binding (NSB, background binding for samples without aptamer). To work under optimum conditions it is very important to control the contact time between the sample mixture and the solid phase. Flow rate should be carefully set up to guarantee that contact time is less than 0.5s. At that contact time, equilibrium is not allowed, therefore, dissociation of aptamer-OA complex is not significant and measured signal is only dependent on free aptamer in solution. Labelled-primers were injected to evaluate the specificity of the aptamer towards immobilised OA. The signal for both approaches after the injection of labelled-primer was lower than 0.2 % of the signal measured for the same concentration of aptamer, thus demonstrating that non-specific adsorption was negligible in front of the interaction due to the affinity of the aptamer for the immobilised OA. Then the aptamer concentration was fixed at 31.5nM to perform equilibrium experiments with different free OA concentrations (Figure 5B). It is important to have an idea of the expected $K_D$ (in the nM range) to determine the range of OA concentrations leading to $K_D$-controlled curves. Data fitting to a standard 1:1 equilibrium model yielded $K_D$ values of 7.4nM and 2.9nM for OA-BSA and biotinylated OA approach, respectively. Table 1 summarises the results for the non-linear four parameter logistic regression fitting of the plots obtained for both KinExA approaches to determine OA in solution, proving the ability of OA aptamer to work as biorecognition element in a KinExA-based sensing assay (n=3).

**Table 1**

| **Strategy** | **$IC_{50}$** ($\mu g \cdot L^{-1}$) | **LOD** ($\mu g \cdot L^{-1}$) | **SD** (%)[a] | **Sigmoidal logistic equation** | **R** |
|---|---|---|---|---|---|
| OA-BSA | 1.8 | 0.3 | 3.8 | $\%binding = -4.7 + \left( \frac{113}{1 + \left( \frac{[OTA]}{1.6} \right)^{0.6}} \right)$ | 0.9996 |
| Biotinylated OA | 28 | 15 | 8.9 | $\%binding = -1.3 + \left( \frac{101}{1 + \left( \frac{[OTA]}{28} \right)^{2.4}} \right)$ | 0.9995 |

[a] *SD values refer to the maximum SD values found in the calibration curve*

OA-BSA approach showed a 50% inhibition of binding ($IC_{50}$= 1.8$\mu$g•$L^{-1}$) lower than that obtained by the biotinylated OA assay ($IC_{50}$= 28$\mu$g• $L^{-1}$). An 80% binding was considered as a limit of detection to be safe in the screening

being 50-fold lower for the OA-BSA-based assay (1.8μg•$L^{-1}$). Low limits of detection (LODs), good reproducibility (maximum standard deviations of 3.8 or 8.9% for OA-BSA- and biotinylated OA-based assay respectively) and the simplicity of the system that can run automatically, show the ability of KinExA to incorporate aptamers as biorecognition elements for sensing applications.

**Example 4: Characterization of OA binding aptamers by Surface Plasmon Resonance**

*Surface Plasmon Resonance (SPR) protocol*

SPR-based technology is commonly used to study the characterisation of real-time biomolecular interactions (Figure 6A) (Drake et al., 2004). SPR measurements were performed with a Biacore T100 system (GE Healthcare) and obtained sensorgrams were analysed using BIAevaluation and Scruber software to determine the dissociation constant ($K_D$). The strategy was based on the immobilisation of OA-BSA conjugate onto CM5 sensor chips. CM5 sensor chips have four different channels. The first channel, modified with BSA, was used as a reference for non-specific adsorptions. The second and forth channels were modified with OA-BSA conjugate, while the third channel was led unmodified. Optimisation of several parameters was performed to improve accuracy and reliability of the $K_D$ determination. Sensor surface modification was performed through covalent binding between the amino groups of the BSA and the carboxylic groups on the sensor surface. Carboxylic groups were activated by a mixture of EDC and NHS, enabling the formation of covalent amide bonds between the carboxylated surface and the BSA molecules. 3μM BSA or OA-BSA solutions prepared in 10mM acetate buffer, pH 4.0, were injected during 30s at a flow rate of 5μL•$min^{-1}$. Afterwards, ethylenediamine solution was let to flow over the sensor surface in order to block all accessible activated carboxyl groups. For each experiment, eleven aptamer concentrations (from 1nM to 100nM) were randomly injected over the biosensor surface in duplicate at 30μL•$s^{-1}$. Several buffer blanks were injected intermittently along an experiment for double referencing. Each sample and blank was injected for 120s. Dissociation was also followed for 120s. An amount of aptamer proportional to the injected aptamer concentration bound the immobilised OA-BSA, resulting in a resonance angle shift. Regeneration of the sensor surface was performed by injecting 1M NaCl in 2x selection buffer during 30s at a flow rate of 30μL•$s^{-1}$. For comparative purposes, all Biacore kinetic experiments were conducted at 25°C and at 4°C. Sensorgrams were processed. Bulk refractive index changes were removed by substracting the reference flow cell (channel 1). Data from all flow cells were globally fit to a 1:1 bimolecular binding model that included a mass transport term to calculate $K_D$ value using BIAevaluation software.

*Results*

Optimisation studies of reagents concentration, flow rate, injection time and regenerating agent, were performed in order to determine the optimal working conditions. Low OA-BSA density on the sensor surface was chosen to prevent steric hindrance, aggregation, rebinding and mass transport limitations. Also to minimise mass transport effects, favoured by the slow diffusion coefficient of the aptamer, relatively high flow rates were used (Karlsson et al., 1994). The SPR data confirmed the affinity of the selected aptamer towards OA previously showed by microtiter plate assays. After aptamer injection, the SPR response progressively increased, while after filling the cell with buffer during the dissociation phase, the SPR response did not return to the baseline level. SPR sensorgrams corresponding to the aptamer's binding performance at 4°C are shown in Figure 3. Global fitting of the association-dissociation curves to a 1:1 Langmuir binding model enabled the calculation of rate and binding constants. The association ($k_a$) and dissociation rate ($k_d$) were determined as $2.7x10^5 M^{-1}s^{-1}$ and $1.3x10^{-3}$ $s^{-1}$, respectively. A $K_D$ value of 4.8nM was obtained based on kinetic data analysis (the standard statistical measure of the closeness of fit, $X_2$ value, was 0.185). The $K_D$ determined for the assay performed at 25°C was 3.8nM. SPR analysis showed that the association of aptamer to immobilised OA was fast and OA-aptamer complex was stable as indicated by a quite slow dissociation rate. Nevertheless, regeneration with 1M NaCl in 2x selection buffer was enough to elute the aptamer from the sensor surface.

*Conclusion*

Since all $K_D$ values found using ELONA, SPR and KinExA were quite close, we can conclude that dissociation of OA-aptamer complex is not so slow and no important artefacts are introduced using ELONA and KinExA measurements. These results confirm that optimum conditions were chosen for ELONA and KinExA measurements to ensure the equilibrium state and the proper assay performance in solution, respectively.

Finally, the selected OA aptamer shows a similar affinity towards OA to that offered by antibodies, but with some additional advantages. The main advantage of aptamers is related to their stability during long-term storage and also under conditions that cause denaturation of antibodies (extreme pH or temperature values, high levels of organic solvents), thus enabling their use under non-physiological conditions and an easy regeneration without loss of activity

(by addition of a chaotropic agent to break the aptamer-target complex). Aptamers can be easily and cost-effectively synthesised, allowing chemical modifications, such as biotinylation or conjugation with fluorophores, without loss of activity. The high affinity of the selected aptamer, with a $K_D$ similar to those of monoclonal antibodies (in the nM range), showed it as a promising alternative biorecognition element.

**Example 5: Optimisation of a competitive ELONA on ELISA microplates**

To prove the ability of the selected aptamer to be used as biorecognition element in sensing assays, a competitive ELONA was optimised. Buffer pH, ionic strength, presence of magnesium and temperature of the competition step, strongly affect the binding capacity of the selected aptamer. Therefore, all these parameters were optimised to provide the best conditions yielding high affinity. Saturated coating of ELISA wells was achieved using a 30nM solution of OA-BSA conjugate. The concentration of biotinylated aptamer was also optimised to 20nM. Different buffers and buffer pH values were assayed, showing that aptamer binding was more efficient at acidic pH. 0.1M acetate buffer, pH 4.5, was chosen for the competition step. This result suggested that the aptamer showed a higher affinity towards protonated OA, likely because OA linked to magnetic beads through its carboxylic group was used for the selection of the aptamer. Moreover, acidic pH probably increased electrostatic interactions between the aptamer and OA. Presence of $MgCl_2$ was also studied, yielding the best binding with 2.5mM $Mg^{2+}$. Divalent ions, such as $Mg^{2+}$, favour the appropriate three-dimensional conformation of the aptamer (Wiegand et al., 1996; Liss et al., 2002). Regarding the temperature of the competition step, better performance was found when incubating at 4°C. The optimal concentration of streptavidin-HRP conjugate, used in an additional step to label the immobilised aptamer, was 0.1mg•$L^{-1}$. The non-specific adsorption of the labelled-streptavidin on bare wells was lower than 0.1%. Results of competitive ELONA were characterised with the assay midpoint, $IC_{50}$, corresponding to the concentration causing a 50% decrease in the assay signal as compared to the signal without competitor. Standard curves from the raw data were fitted using a four-parameter sigmoidal equation. Under optimum conditions, $IC_{50}$ was determined to be 4.1nM (approx. 3.3$\mu$g•$L^{-1}$), proving the ability of the selected aptamer to be implemented in a sensing assay to assess OA under the limit set by the European Commission (160$\mu$g•$kg^{-1}$) (Commission Regulation (EC) No. 853/2004).

**Example 6: Optimisation of a competitive ELONA with OA-modified magnetic beads**

A colorimetric assay using ELONA was developed as an indirect competitive assay with OA-modified magnetic beads, to minimise the non-specific adsorptions oberved for the indirect competitive assays performed on microtiter plates. A fixed concentration of ALP-modified aptamer (Eurogentec) was used in the competition step. The non-linear four parameter logistic regression fitting of the plot showed a 50% inhibition of binding of 2.3nM (1.8$\mu$g$L^{-1}$) (Figure 4B). ELONA competitive assay shows $IC_{50}$ values of less than 10 $\mu$g•$L^{-1}$, proving the ability of the selected aptamer to be implemented in sensing assays to assess OA under the limits set by the European Commission.

**Example 7: Indirect competitive Kinetic Exclusion Assay based on OA-BSA or biotinylated OA-modified PMMA beads**

KinExA measurements for OA detection were performed using two different indirect competitive approaches based on OA-BSA or biotinylated-BSA conjugate immobilised on the PMMA beads used as solid phase (Figure 5A). Competition was performed using optimized conditions as set in Example 3, with a fixed concentration of FITC-labelled aptamer of 31,5 nM. and different OA concentrations in solution. OA-BSA approach showed a 50% inhibition of binding ($IC_{50}$ = 1.8$\mu$g$L^{-1}$) lower than that obtained by the biotinylated OA-based assay ($IC_{50}$ = 28$\mu$g$L^{-1}$) (Figure 5B). A 10% fluorescence was considered as a limit of detection to be safe in the screening being 0.3 and 15$\mu$g$L^{-1}$ for OA-BSA and biotinylated OA-assays, respectively. KinExA competitive assay shows $IC_{50}$ values of less than 10 $\mu$g•$L^{-1}$, proving the ability of the selected aptamer to be implemented in sensing assays to assess OA under the limits set by the European Commission.

**Example 8: SPR for direct OA detection using aptamer-modified chips**

SPR was used to study the effect of different modifications at the 5'end of the aptamer towards OA detection. A CM5 gold chip was modified by self-assembling of a thiolated short oligonucleotide complementary to the 3' end of the aptamer (Figure 6A). Hybridization of the aptamer to a previously immobilized short oligonucleotide complementary to one of the ends of the aptamer was performed according to a simple protocol widely used in biosensor preparation, based on the initial formation of a self-assembled monolayer (SAM) by chemisorption of a thiolated single strand DNA sequence, followed by hybridization of a complementary single strand DNA sequence, as described for example in Tonga (2011). This was followed by hybridisation with aptamer, aptamer-biotin or aptamer-ALP (Eurogentec). An oligonucleotide of a similar length to the OA aptamer was hydridised on the control channel. Results, after subtraction of the response for the control channel, showed that both labelled aptamers, biotinylated and ALP-aptamer, offer higher RU values than those for the unlabelled aptamer (negative values for all the approaches could be explained based on the aptamer dehybridisation or conformational changes caused by the presence of OA). Therefore, assays based on labelled aptamers are more sensitive. Figure 6A shows a scheme of the SPR-based aptasensing system used to detect OA and the obtained calibration curves using OA-BSA- and biotinylated OA-modified PMMA beads. These approaches enabled the detection of OA concentrations as low as 0.1nM ($81 ngL^{-1}$) (Figure 6B).

**Example 9: Extraction procedure of OA from spiked mussels**

*Protocol 1:* Mussels were rinsed with water and opened by cutting the adductor muscle with a scalpel. Homogenisation of the whole flesh was performed using a handheld blender. Extraction was performed with 10mL methanol vortexed for 5 s and mixed during 30 min. Crude extracts were centrifuged at 8000rpm and 10°C for 10 min. Collected supernatants were evaporated to dryness under nitrogen at room temperature. The residues were resuspended in 1mL PBS and the reconstituted solutions were passed through 0.45-μm cut-off Whatman nylon membrane filters. Scallop and oyster samples were prepared following a similar procedure.

If necessary, a purification step of extracts may be performed so as to reduce or eliminate the presence of some organic or inorganic compounds components possibly interfering with the detection. Said compounds may be pigments in particular, such as pigments conferring a yellow/orange color of mussels. Purification steps can be performed according to methods known by a person skilled in the art.

*Protocol 2*: In the presence of OA covalently linked to shellfish matrix, a first step of hydrolysis was performed as following: 9 mL of methanol was added to 4 g of shellfish tissue and the mixture was mixed with a vortex for 2 min before being centrifuged at 2000 × g for 10 min at room temperature. The supernatant was poured into a flask and the solution was completed with distilled water up to 10 mL. For hydrolysis, 1 mL of mussel extract was poured into a tube and 136 μL of 2.5N NaOH was added. The tube was then placed at 74 °C for 40 min. The solution was cooled down to room temperature and neutralized by adding 108 μL of 2.5N HCl. Controls were achieved by spiking crude methanol extract with the same amounts of OA. Different blanks were also achieved to eliminate the matrix effect and the inhibitory effect of methanol.

*Protocol 3*: Each gram of whole oyster (or of whole mussel) in homogenized with 4 to 6 (preferably 6) ml of methanol/deionized water (80/20) at room temperature. The mixture is centrifuged for 10 mn at 3000g and the supernatant is collected. 2 ml of methanol/deionized water 80/20 is added to tissue residue, the mixture is centrifuged for 10 mn at 3000g and the supernatant is added to the first portion. The final volume is brought to 10 ml with methanol/deionized water (80/20). The extract is then filtered through 0,45 microm filter. 10 microL are removed from the extract and diluted to 10 ml with the sample dilution buffer (dilution 1/100). Highly contaminated samples can be diluted further and re-analyzed. Samples with low concentration of OA may be analyzed at lesser dilutions.

Example 10: Detection of OA in shellfish samples by competitive ELONA

Oyster samples were prepared according to protocol 1 described in Example 9. The detection protocol is performed according to the protocol of example 5. Exact quantitation of assayed OA is achieved by adapting the dilution of the biological sample such that the degree of inhibition falls within a standard inhibition curve obtained with known amounts of OA. OA coated plates prepared as described in example 5 were contacted with 100μL per well of serial dilutions of the sample in 0.1M acetate buffer, pH 4.5. Biotinylated aptamer 20nM in 0.1M acetate buffer, pH 4.5, 2.5mM $Mg^{2+}$, was added. Dilution factors used are comprised between 1/10 (+/- 10%) and 1/10 000 (+/-10%). Plates were incubated for 1h. Streptavidin-HRP conjugate $0.1 mg \cdot L^{-1}$ solutions was added. 100μL of HRP substrate solution (TMB liquid substrate) was then incubated. Absorbance values were measured at 620nm. Each step was performed at room temperature with shaking and protected from light. The wells were thoroughly rinsed three times with 0.1M PBS containing 0.05% Tween 20 (PBS-T) between each step. Assays were performed in triplicate.

Results of competitive ELONA were characterised with the assay midpoint, $IC_{50}$, corresponding to the concentration causing a 50% decrease in the assay signal as compared to the signal without competitor. Standard curves from the raw data were fitted using a four-parameter sigmoidal equation. Comparison of data obtained in the presence of shellfish sample with standard curves allows determination of the presence and concentration of OA in said samples.

REFERENCES

Barthelmebs, Jonca, Hayat, Prieto-Simon, Marty, 2011, Food Control 22, 737-743.

Blake II, R.C., Pavlov, A.R., Blake, D.A., 1999. Anal. Biochem. 272, 123-134.
Campàs, M., de la Iglesia, P., Le Berre, M., Kane, M., Diogène, J., Marty, J.-L., 2008.Biosens. Bioelectron. 24, 716-722.
Campàs, M., Marty, J.-L., 2007. Anal. Chim. Acta 605, 87-93.
Campàs M., Reverté L., Freixes G., de la Iglesia P., Diogène J., Prieto-Simón B., (2011) P131, 22nd International Conference on Bio-Sensing Technology 2011, 10-12 October 2011, Amsterdam
Commission Regulation (EC) No. 853/2004 of 29 April 2004. Off. J. Eur. Communities L 139/55 (current as of May 2008 at http://eur-lex.europa.eu/en/index.htm).
Commission Regulation (EC) No. 2074/2005 of 5 December 2005. Off. J. Eur. Communities L 338/27-59 (current as of May 2008 at http://eur-lex.europa.eu/en/index.htm).
Drake, A.W., Myszka, D.G., Klakamp, S.L., 2004. Anal. Biochem. 328, 35-43.
Ellington, A.D., Szostak, J.W., 1990. Nature 346, 818-822.
Hayat A., Barthelemebs 1., Sassolas A., Marty JL., 2012, Anal. Chim. Acta, 724: 92-7
Karlsson, R., Roos, H., Fägerstam, L., Persson, B., 1994. Methods Enzymol. 6, 99-110.
Khosraviani, M., Blake II, R.C., Pavlov, A.R., Lorbach, S.C., Yu, H., Delehanty, J.B., Brechbiel, M.W., Blake, D.A., 2000. Bioconjug. Chem. 11, 267-277.
Kreuzer, M.P., Pravda, M., O'Sullivan, C.K., Guilbault, G.G., 2002. Toxicon 40, 1267-1274.
Levine, L., Fujiki, H., Yamada, K., Ojika, M., Gjika, H.B., Van Vunakis, H., 1988. Toxicon 26, 1123-1128.
Liss, M., Petersen, B., Wolf, H., Prohaska, E., 2002. Anal. Chem. 74, 4488-4495.
Llamas, N.M., Stewart, L., Fodey, T., Higgins, H.C., Velasco, M.L.R., Botana, L.M., Elliott, C.T., 2007. Anal. Bioanal. Chem. 389, 581-587.
Loomans, E.E.M.G., Roelen, A.J.M., Van Damme, H.S., Bloemers, H.P.J., Gribnau, T.C.J., Schielen, W.J.G, 1995. J. Immunol. Methods 184, 207-217.
Marquette, C.A., Coulet, P.R., Blum, L.J., 1999. Anal. Chim. Acta 398, 173-182.
Prieto-Simón, B., Miyachi H., Karube, I., Saiki, H., 2010. Biosens. Bioelectron. 25, 1395-1401.
Stoltenburg R., Reinemann C., Strehlitz B., 2005. Anal. Bioanal. Chem. 383, 83-91.
Stoltenburg R., Reinemann C., Strehlitz B., 2007. Biomol. Engineering 24, 381-403.
Tang, A.X.J., Pravda, M., Guilbault, G.G., Piletsky, S., Turner, A.P.F., 2002. Anal. Chim. Acta 471, 33-40.
Tonga, L. Zhang, J.-J. Xub, H.-Y. Chen. ,2011, Biosensors and Bioelectronics 29, 97-101.
Tuerk, C., Gold, L., 1990. Science 249, 505-510.
Wiegand, T.W. et al., 1996. J. Immunol. 157, 221-230.
Yang et al., Biosens. Bioelectron., 2012, Feb 15;32(1): 208-12.
European patent applications N° 0 311 456 and N° 0 509 819
US patent N°8,180,665

## Claims

1. Isolated nucleic acid compound able to specifically bind to okadaic acid, **characterized in that** it comprises a nucleotide sequence of at least 30 nucleotides, said sequence having at least 80% identity, preferably at least 90% identity, more preferably at least 95% identity and even more preferably at least 98% identity with SEQ ID N°3.

2. Nucleic acid compound according to claim 1 wherein said nucleic acid compound is an aptamer.

3. Nucleic acid compound according to claim 1 or 2, wherein said nucleic acid comprises a sequence selected from the group consisting of: SEQ ID N°3, functional analogues thereof and functional fragments thereof.

4. Nucleic acid compound according to any one of claim 1 to 3, wherein the sequence of said nucleic acid is SEQ ID N°3.

5. Nucleic acid compound according to any one of claim 1 to 4, wherein said compound is chemically coupled to a labeling element.

6. A method for detecting okadaic acid in a sample comprising the steps of:

   (a) providing a sample,
   (b) contacting said sample with an isolated nucleic acid according to any one of claims 1 to 5, under conditions wherein an okadaic acid/nucleic acid complex may be formed; and
   (c) determining whether said okadaic acid/nucleic acid complex is formed, thereby determining the presence of okadaic acid in said sample.

7. A method for determining the concentration of okadaic acid in a sample comprising the steps of:

   (a) providing a sample,
   (b) contacting said sample with an isolated nucleic acid according to any one of claims 1 to 5, under conditions wherein an okadaic acid/nucleic acid complex may be formed; and
   (c) quantitatively detecting the okadaic acid/nucleic acid complex, thereby determining the concentration of the okadaic acid in said sample.

8. The method according to claim 6 or 7, wherein said sample is a biological sample obtained from a shellfish selected from the group consisting of: mussels, scallops, oysters and clams,

9. The method according to any one of claims 6 to 8, wherein said method comprises a competitive assay chosen among the following: enzyme-linked oligonucleotide assay (ELONA) and kinetic exclusion assay (KinExA).

10. The method according to any one of claims 6 or 7, wherein said method comprises a direct detection of the okadaic acid/nucleic acid complex.

11. The method according to any one of claims 6 to 10, wherein said contacting step is performed in the presence of an organic solvent chosen among the group consisting of: methanol, dimethyl sulfoxide, ethanol, and acetonitrile.

12. The method according to any one of claims 6 to 11 wherein said contacting step is performed at a pH value comprised between about pH 4 and about pH 5, and preferably at a pH value of about pH 4,5.

13. A kit for the detection of okadaic acid in a sample, comprising at least a nucleic acid compound according to any one of claims 1 to 5.

14. A device for the detection of okadaic acid in a sample, comprising at least a nucleic acid compound according to any one of claims 1 to 5, immobilized on a solid phase support.

15. Use of an isolated nucleic acid compound according to any one of claims 1 to 5, for the detection of the presence of okadaic acid in a biological sample or for the determination of the concentration of okadaic acid in a biological sample.

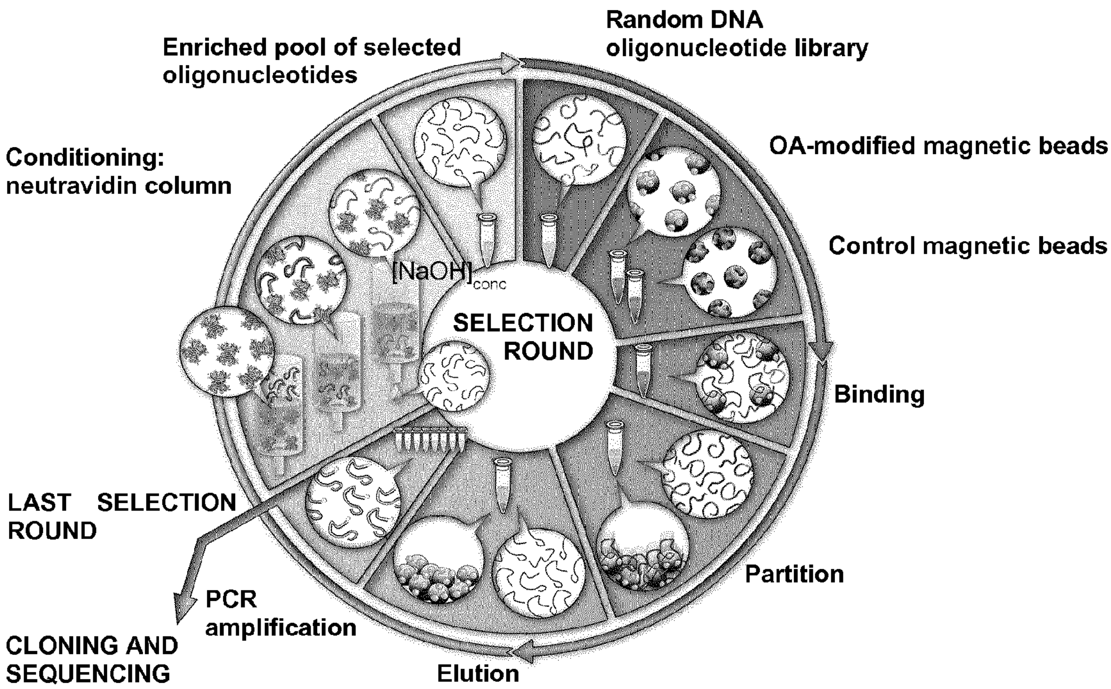


Figure 1

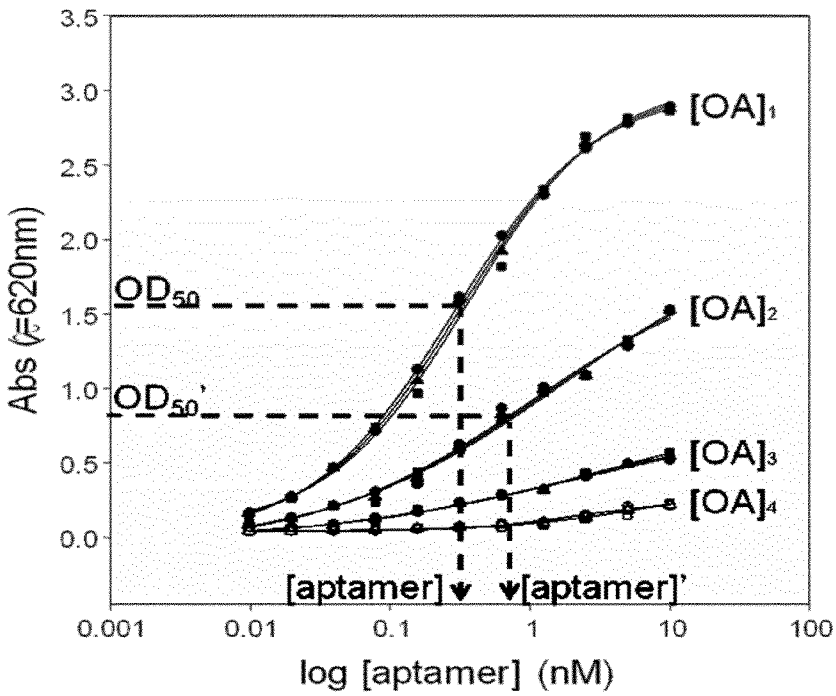


Figure 2

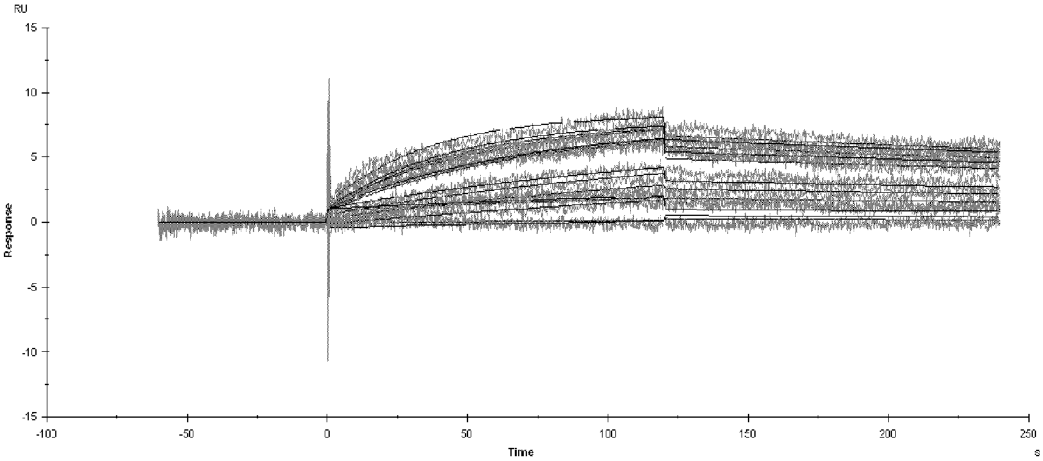


Figure 3

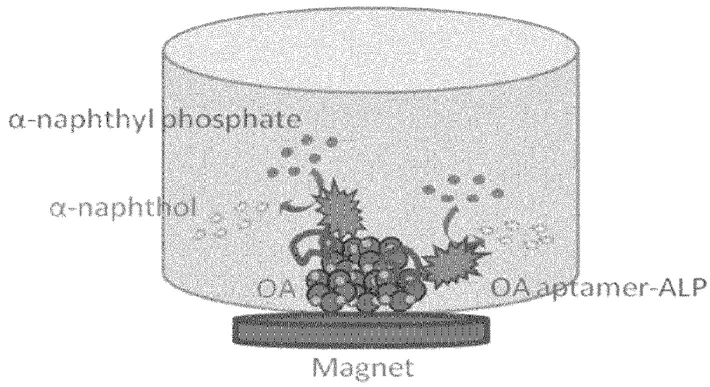


Figure 4A

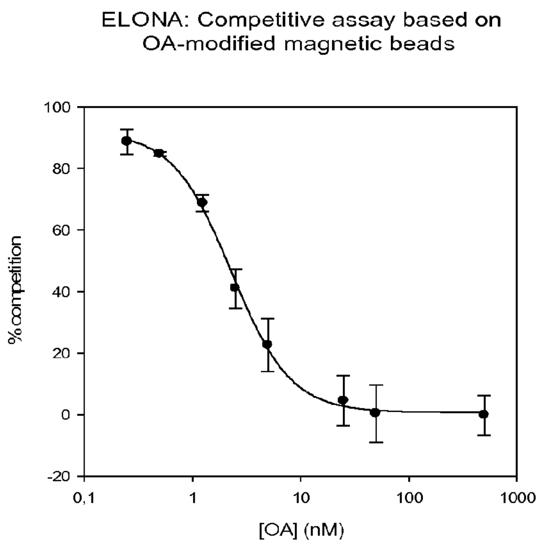


Figure 4B

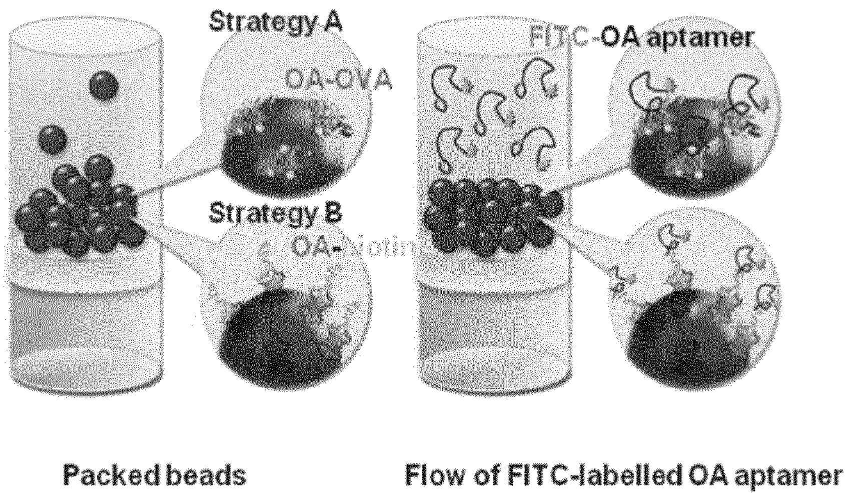


Figure 5A

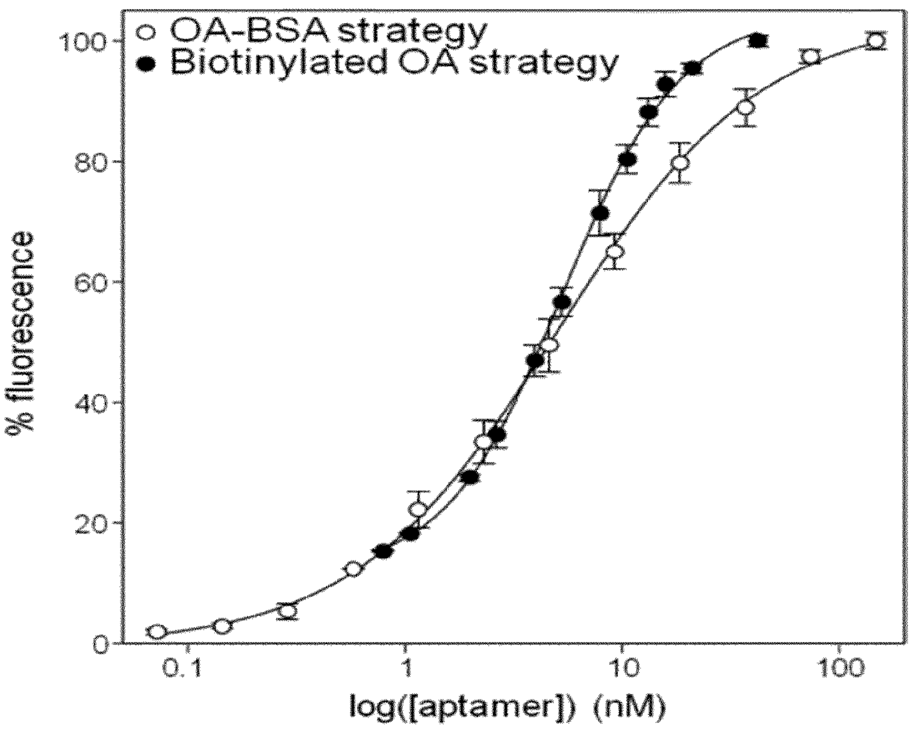


Figure 5B

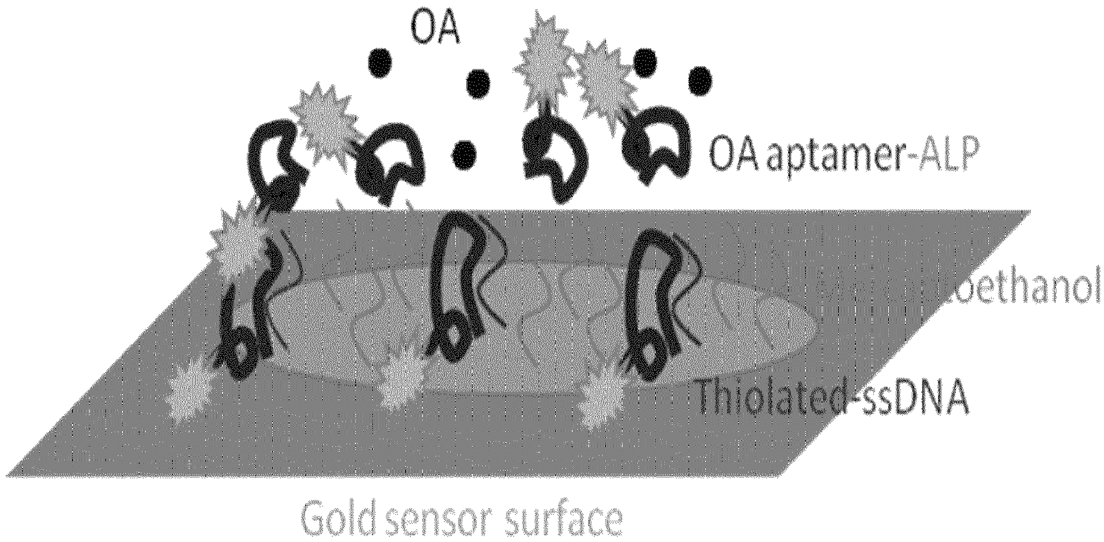


**Figure 6A**

SPR: direct assay based on aptamer-modified gold chips

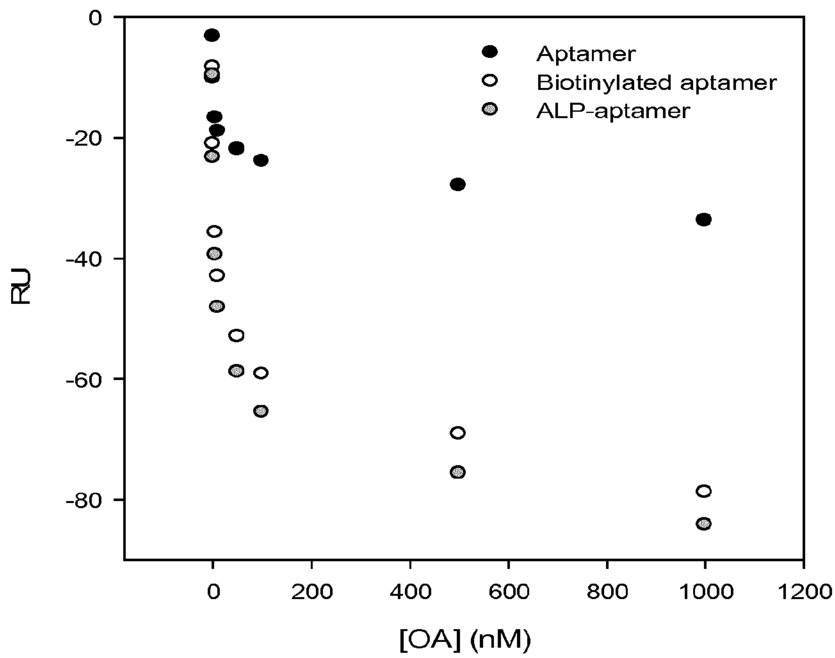


**Figure 6B**

Europäisches Patentamt
European Patent Office
Office européen des brevets

# EUROPEAN SEARCH REPORT

Application Number
EP 13 30 5214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AUDREY SASSOLAS ET AL: "Detection of the marine toxin okadaic acid: Assessing seafood safety", TALANTA, vol. 105, 1 February 2013 (2013-02-01), pages 306-316, XP055069099, ISSN: 0039-9140, DOI: 10.1016/j.talanta.2012.10.049 * the whole document * ----- | 1-15 | INV. C12N15/115 C12Q1/68 G01N33/53 |
| A | AKHTAR HAYAT ET AL: "Development of a novel label-free amperometric immunosensor for the detection of okadaic acid", ANALYTICA CHIMICA ACTA, vol. 724, 1 April 2012 (2012-04-01), pages 92-97, XP055069084, ISSN: 0003-2670, DOI: 10.1016/j.aca.2012.02.035 * the whole document * ----- | 1-15 | |
| A | MÒNICA CAMPÀS ET AL: "Novel nanobiotechnological concepts in electrochemical biosensors for the analysis of toxins", THE ANALYST, vol. 137, no. 5, 11 January 2012 (2012-01-11), page 1055, XP55069129, ISSN: 0003-2654, DOI: 10.1039/c2an15736e * the whole document * ----- -/-- | 1-15 | TECHNICAL FIELDS SEARCHED (IPC): C12N C12Q G01N |

The present search report has been drawn up for all claims

1

| Place of search | Date of completion of the search | Examiner |
|---|---|---|
| The Hague | 3 July 2013 | Andres, Serge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 5214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CHENG YANG ET AL: "Aptamer-DNAzyme hairpins for biosensing of Ochratoxin A", BIOSENSORS AND BIOELECTRONICS, vol. 32, no. 1, 5 December 2011 (2011-12-05), pages 208-212, XP028440359, ISSN: 0956-5663, DOI: 10.1016/J.BIOS.2011.12.011 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 3 July 2013 | Andres, Serge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0311456 A
- EP 0509819 A
- US 5180665 A
- US 8180665 B


### Non-patent literature cited in the description


- **STOLTENBURG.** *SELEX technology FluMag-SELEX,* 2005
- **BARTHELMEBS ; JONCA ; HAYAT ; PRIETO-SIMON ; MARTY.** *Food Control,* 2011, vol. 22, 737-743
- **BLAKE II, R.C. ; PAVLOV, A.R. ; BLAKE, D.A.** *Anal. Biochem.,* 1999, vol. 272, 123-134
- **CAMPÀS, M. ; E LA IGLESIA, P. ; LE BERRE, M. ; KANE, M. ; DIOGÈNE, J. ; MARTY, J.-L.** *Biosens. Bioelectron,* 2008, vol. 24, 716-722
- **CAMPÀS, M. ; MARTY, J.-L.** *Anal. Chim. Acta,* 2007, vol. 605, 87-93
- **CAMPÀS M. ; REVERTÉ L. ; FREIXES G. ; DE LA IGLESIA P. ; DIOGÈNE J. ; PRIETO-SIMÓN B.** *22nd International Conference on Bio-Sensing Technology,* 10 October 2011, 131
- **DRAKE, A.W. ; MYSZKA, D.G. ; KLAKAMP, S.L.** *Anal. Biochem.,* 2004, vol. 328, 35-43
- **ELLINGTON, A.D. ; SZOSTAK, J.W.** *Nature,* 1990, vol. 346, 818-822
- **HAYAT A. ; BARTHELEMEBS 1. ; SASSOLAS A. ; MARTY JL.** *Anal. Chim. Acta,* 2012, vol. 724, 92-7
- **KARLSSON, R. ; ROOS, H. ; FÄGERSTAM, L. ; PERSSON, B.** *Methods Enzymol.,* 1994, vol. 6, 99-110
- **KHOSRAVIANI, M. ; BLAKE II, R.C. ; PAVLOV, A.R. ; LORBACH, S.C. ; YU, H. ; DELEHANTY, J.B. ; BRECHBIEL, M.W. ; BLAKE, D.A.** *Bioconjug. Chem.,* 2000, vol. 11, 267-277
- **KREUZER, M.P. ; PRAVDA, M. ; O'SULLIVAN, C.K. ; GUILBAULT, G.G.** *Toxicon,* 2002, vol. 40, 1267-1274
- **LEVINE, L. ; FUJIKI, H. ; YAMADA, K. ; OJIKA, M. ; GJIKA, H.B. ; VAN VUNAKIS, H.** *Toxicon,* 1988, vol. 26, 1123-1128
- **LISS, M. ; PETERSEN, B. ; WOLF, H. ; PROHASKA, E.** *Anal. Chem.,* 2002, vol. 74, 4488-4495
- **LLAMAS, N.M. ; STEWART, L. ; FODEY, T. ; HIGGINS, H.C. ; VELASCO, M.L.R. ; BOTANA, L.M. ; ELLIOTT, C.T.** *Anal. Bioanal. Chem.,* 2007, vol. 389, 581-587
- **LOOMANS, E.E.M.G. ; ROELEN, A.J.M. ; VAN DAMME, H.S. ; BLOEMERS, H.P.J. ; GRIBNAU, T.C.J. ; SCHIELEN, W.J.G.** *J. Immunol. Methods,* 1995, vol. 184, 207-217
- **MARQUETTE, C.A. ; COULET, P.R. ; BLUM, L.J.** *Anal. Chim. Acta,* 1999, vol. 398, 173-182
- **PRIETO-SIMÓN, B. ; MIYACHI H. ; KARUBE, I. ; SAIKI, H.** *Biosens. Bioelectron.,* 2010, vol. 25, 1395-1401
- **STOLTENBURG R. ; REINEMANN C. ; STREHLITZ B.** *Anal. Bioanal. Chem.,* 2005, vol. 383, 83-91
- **STOLTENBURG R. ; REINEMANN C. ; STREHLITZ B.** *Biomol. Engineering,* 2007, vol. 24, 381-403
- **TANG, A.X.J. ; PRAVDA, M. ; GUILBAULT, G.G. ; PILETSKY, S. ; TURNER, A.P.F.** *Anal. Chim. Acta,* 2002, vol. 471, 33-40
- **TONGA, L. ; ZHANG, J.-J. XUB ; H.-Y. CHEN.** *Biosensors and Bioelectronics,* 2011, vol. 29, 97-101
- **TUERK, C. ; GOLD, L.** *Science,* 1990, vol. 249, 505-510
- **WIEGAND, T.W. et al.** *J. Immunol.,* 1996, vol. 157, 221-230
- **YANG et al.** *Biosens. Bioelectron.,* 15 February 2012, vol. 32 (1), 208-12